# EUROPEAN PATENT APPLICATION

(11) **EP 4 636 778 A1**
(43) Date of publication of application: **22.10.2025**
(21) Application number: 24203199.5
(22) Date of filing: 27.09.2024
(51) Int. Cl.: G16H 30/40

(54) **A METHOD FOR GENERATING PATIENT MOTION DATA FOR A MEDICAL IMAGING EXAMINATION OF A PATIENT**

(71) Applicant: Siemens Healthineers AG, 91301 Forchheim (DE)
(72) Inventor: Kang, Henry, 91052 Erlangen (DE); Coutinho, Mariana, 91056 Erlangen (DE)
(74) Representative: Siemens Healthineers Patent Attorneys

(57) **Abstract**

The invention relates in one aspect to a computer-implemented method for generating patient motion data for a medical imaging examination of a patient, the method comprising:
- receiving input data, the input data comprising information regarding a facial action of the patient,
- applying a trained machine learning model to the input data, wherein the patient motion data is generated based on the input data, the patient motion data comprising information regarding a motion of a non-facial body part of the patient, wherein the information regarding the motion of the non-facial body part of the patient is generated based on the information regarding the facial action of the patient.

## Description

In one aspect the invention relates to a computer-implemented method for generating patient motion data for a medical imaging examination of a patient. In other aspects, the invention relates to a data processing system, a medical imaging system, a computer program product and a computer-readable storage medium.

During medical imaging using, for example, computed tomography or magnetic resonance, patient motions may occur that create blurs or other artifacts in the processed images. Motion intelligence (Al). However, due to technical limitations of the artifact reduction, the motions may still have a negative impact on clinical outcomes, sometimes to the extent that a repeat of the medical imaging examination may be necessary.

Patients often exhibit certain behavioural patterns, for example, frequent movement due to pain or anxiety. Due to lack of time and high effort necessary for communications by the medical professionals, such early indicators can easily be overlooked. Basically, medical professionals may check through oral communication if the patient is feeling well enough to decide, if the patient would be able to comply with the requirements (e.g. holding movements, controlling breathing, etc.) during scanning.

DE 10 2020 213 690 A1 discloses a method for setting a shape of a headrest based on radiological image data and optical image data relating to a head of a patient.

US 11 730 440 B2 discloses a method for controlling a medical imaging examination of a subject based on motion tracking analysis.

The underlying technical problem of the invention is to facilitate a generating of patient motion data for a medical imaging examination of a patient. This problem is solved by the subject matter of the independent claims. The dependent claims are related to further aspects of the invention. Independent of the grammatical term usage, individuals with male, female or other gender identities are included within the term.

In one aspect the invention relates to a computer-implemented method for generating patient motion data for a medical imaging examination of a patient, the method comprising:
- receiving input data, the input data comprising information regarding a facial action of the patient,
- applying a trained machine learning model to the input data, wherein the patient motion data is generated based on the input data, the patient motion data comprising information regarding a motion of a non-facial body part of the patient, wherein the information regarding the motion of the non-facial body part of the patient is generated based on the information regarding the facial action of the patient.

In particular, the facial action of the patient may be a facial movement and/or facial expression of a face of the patient. The facial action may be an action of an individual facial muscle or an action of a group of facial muscles. In particular, the facial action may be an individual fascial muscle movement or a group of facial muscle movements. The facial muscle movements of the group of facial muscle movements may relate to different facial muscles. Each facial muscle movement of the group of facial muscle movements may be, for example, a contraction or a relaxation of the related facial muscle. The facial action may be, for example, emotion related. The facial action may be, for example, an indicator of the patient's anxiety, pain or any discomfort.

The input data may comprise the information regarding the facial action of the patient, for example, in form of a video and/or a sequence of images showing the facial action of the patient and/or in form of a facial action code, in particular an alphanumeric facial action code, representing the facial action of the patient. In particular, the trained machine learning model may be applied to the input data, thereby generating the patient motion data based on the input data and/or generating the information regarding the motion of the non-facial body part of the patient based on the information regarding the facial action of the patient.

In particular, the information regarding the motion of the non-facial body part of the patient may be generated even if the non-facial body part of the patient cannot be seen, for example, by an operator or a camera system. In particular, the information regarding the motion of the non-facial body part of the patient may be generated while the non-facial body part of the patient is covered from visual observation, for example, due to clothing, radiation shielding or medical accessories, and/or while the non-facial body part of the patient is located inside an examination area of a medical imaging device.

The motion of the non-facial body part of the patient may be, for example, a motion of the non-facial body part of the patient relative to a part of a patient handling system and/or relative to an examination area of a medical imaging device. The motion of the non-facial body part of the patient may be, for example, actively driven by muscles of the patient, in particular by non-facial muscles of the patient. The motion of the non-facial body part of the patient may be, for example, a motion, in particular, a raising, a lowering, a twisting, an extension or a contraction, of the non-facial body part of the patient, for example, relative to a reference body part of the patient and/or relative to an anatomical axis of the patient and/or relative to a system axis of the medical imaging device. The patient motion data may be provided. The patient motion data may be provided, for example, to an operator, in particular through a display, and/or to a control unit of a medical imaging device to adjust the medical imaging examination, in particular to adjust the medical imaging examination manually or automatically.

The medical imaging examination may be, for example, a computed tomography examination or a magnetic-resonance imaging examination. For example, if a motion-related low-quality of a result of the medical imaging examination is predicted with high probability before the medical imaging examination is completed, the medical imaging examination may be interrupted and/or redone immediately. Therefore, healthcare service providers and patients may save time by reducing the need for full image repetitions. This also means less exposure to ionizing radiation in comparison to fully repeated imaging. Furthermore, this enables early intervention during scanning if the patient exhibits critical changes according to the facial action coding system. The image quality may be improved in comparison to current motion correction solutions for motion artifacts as the result of the medical imaging examination that is carried out based on the patient motion data may have less or even no motion artifacts. Therefore, patient and user experience may be improved.

According to a further aspect, camera image data of a face of the patient is received, wherein the input data is generated based on the camera image data. The camera image data may comprise, for example, a camera video and/or a sequence of temporally consecutive camera images. The camera image data may be, for example, 2D camera image data or 3D camera image data. The camera image data may comprise, for example, RGB image data and/or depth data regarding a field of view of a camera system.

According to a further aspect, the input data is generated by applying an algorithm for face detection and/or feature extraction to the camera image data. The algorithm for face detection and/or feature extraction may be, for example, a trained machine learning algorithm and/or a face recognition algorithm. In particular, the face of the patient may be detected, in particular in real-time, by applying the algorithm for face detection and/or feature extraction to the camera image data, in particular to the RGB image data and/or the depth data regarding the field of view of the camera system. In particular, a plurality of facial landmarks may be extracted, in particular in real-time, by applying the algorithm for face detection and feature extraction to the camera image data, in particular to the RGB image data and/or the depth data regarding the field of view of the camera system. The information regarding the facial action of the patient may be calculated, for example, based on the plurality of facial landmarks, in particular based on movements of the facial landmarks of the plurality of facial landmarks. The information regarding the facial action of the patient may be calculated, for example, by applying a motion tracking algorithm, in particular a vector-field-based motion tracking algorithm, on the plurality of facial landmarks.

According to a further aspect, the information regarding the facial action of the patient is indicative of a movement of at least one facial muscle and/or of a movement of at least one facial landmark. According to a further aspect, the information regarding the facial action of the patient is indicative of a facial action unit according to a facial action coding system.

The information regarding the facial action of the patient may be indicative, for example, of an intensity of the facial action unit according to the facial action coding system. The facial action coding system (FACS) may be an anatomically-based taxonomy system, in particular for describing observable facial movements and/or for identifying and categorizing facial expressions by breaking them down into individual components called "action units" (AUs). Each action unit may correspond to the movement of specific facial muscles. This system allows for detailed analysis of facial expressions, which can be useful in various applications such as emotion detection, psychological research, and human-computer interaction.

The information regarding the facial action may be generated, for example, based on a facial action coding model and the plurality of facial landmarks. The facial action coding model may relate, for example, facial action units of the facial action coding system to the movements of the facial landmarks of the plurality of facial landmarks.

According to a further aspect, the information regarding the motion of the non-facial body part of the patient is indicative of a probability that the motion of the non-facial body part of the patient will occur during the medical imaging examination of the patient. Thus, the method could be used to provide, in particular in real-time, a prediction regarding the motion of the non-facial body part of the patient. According to a further aspect, the information regarding the motion of the non-facial body part of the patient is indicative of an impact of the motion of the non-facial body part of the patient on the medical imaging examination of the patient.

In particular, the information regarding the motion of the non-facial body part of the patient may be indicative of whether an intensity of the motion of the non-facial body part of the patient exceeds a corresponding threshold. In particular, the information regarding the motion of the non-facial body part of the patient may be indicative of whether an expected medical imaging artifact intensity related to the motion of the non-facial body part of the patient exceeds a corresponding threshold. Thus, the method could be used to provide, in particular in real-time, a prediction regarding an expected medical imaging artifact related to the motion of the non-facial body part of the patient.

According to a further aspect, the medical imaging examination of the patient by a medical imaging device is adjusted based on the patient motion data. For example, a control signal for the medical imaging device may be calculated based on the patient motion data. In particular, the medical imaging examination may be started and/or interrupted based on the patient motion data and/or based on the control signal. In particular, a parameter of the medical imaging examination may be adjusted based on the patient motion data and/or based on the control signal. The parameter of the medical imaging examination may be, in particular a scan protocol parameter, for example an x-ray source parameter and/or a pitch, and/or an image reconstruction parameter, for example a kernel parameter and/or a noise reduction parameter. For example, an artifact-reduction algorithm for the medical imaging examination may be activated based on the patient motion data. For example, a medical imaging dataset acquired in the medical imaging examination may be flagged based on the patient motion data.

According to a further aspect, the non-facial body part of the patient comprises at least a region of a torso of the patient and/or at least a region of an extremity of the patient. The patient may be a human or an animal. The face of the patient may be a part of a head of the patient. In particular, the non-facial body part of the patient may be distinct from the face. For example, a transverse plane and/or a coronal plane may be located between the face of the patient and the non-facial body part of the patient. The at least a region of the extremity of the patient may be, for example, an arm, a hand, a leg and/or a foot. The non-facial body part of the patient may comprise, for example, a region of a skull of the patient and/or a region of a neck of the patient.

In a further aspect, the invention relates to a data processing system for generating patient motion data for a medical imaging examination of a patient, the data processing system comprising:
- an interface, configured for receiving input data, the input data comprising information regarding a facial action of the patient, and
- a calculation unit, configured for applying a trained machine learning model to the input data, wherein the patient motion data is generated based on the input data, the patient motion data comprising information regarding a motion of a non-facial body part of the patient, wherein the information regarding the motion of the non-facial body part of the patient is generated based on the information regarding the facial action of the patient.

According to a further aspect, the medical imaging system comprises:
- the data processing system according to one of the aspects of the invention,
- a medical imaging device, configured for carrying out the medical imaging examination of the patient,
- a camera system, configured for providing camera image data of a face of the patient,
- a preprocessing unit, configured for generating the input data based on the camera image data.

In particular, the camera image data may be provided by the camera system to the preprocessing unit and/or received by the preprocessing unit from the camera system. In particular, the input data may be provided by the pre-processing unit to the calculation unit and/or received by the calculation unit from the pre-processing unit. The medical imaging device may be, for example, a computed tomography device or a magnetic-resonance imaging device. The medical imaging device may be configured for carrying out the medical imaging examination of the patient. The medical imaging device may be, for example, a computed tomography device, a magnetic-resonance imaging device, an ultrasound imaging device, a PET imaging device, a SPECT imaging device, an X-ray imaging device, a cone beam CT device and/or a combination thereof.

According to a further aspect, the medical imaging system comprises a patient handling system, configured for positioning the patient relative to an examination area of the medical imaging device such that the face of the patient is located in a field of view of the camera system when the non-facial body part of the patient is located in the examination area of the medical imaging device.

The camera system may be configured for acquiring the camera image data of the face of the patient. The camera system may comprise a 2D camera and/or a 3D camera. The camera system may comprise a plurality of 2D cameras and/or a plurality of 3D cameras. In particular, the non-facial body part may be located outside of the field of view of the camera system when the non-facial body part of the patient is located in the examination area of the medical imaging device and/or the non-facial body part may be hidden from the camera system when the non-facial body part of the patient is located in the examination area of the medical imaging device.

In a further aspect, the invention relates to a computer-implemented method for providing a trained machine learning model, the method comprising:
- receiving input training data, the input training data comprising information regarding a facial action of a patient,
- receiving output training data, wherein the output training data is related to the input training data, the output training data comprising information regarding a motion of a non-facial body part of the patient, wherein the information regarding the motion of the non-facial body part of the patient is related to the information regarding the facial action of the patient,
- training a machine learning model based on the input training data and the output training data,
- providing the trained machine learning model.

In particular, the machine learning model may be trained based on the input training data and the output training data to correlate facial actions to motions of non-facial body parts of the patient. The information regarding the motion of the non-facial body part of the patient for the output training data may be derived, in particular manually or automatically derived, for example, from medical images of the non-facial body part of the patient, in particular in form of DICOM images, acquired in corresponding medical imaging examinations. The information regarding the motion of the non-facial body part of the patient for the output training data may be calculated, for example, by applying a motion tracking algorithm, in particular a vector-field-based motion tracking algorithm, on a plurality of landmarks in optical image data and/or in radiological image data of the non-facial body part of the patient.

In particular, the machine learning model can be trained with a plurality of training data sets, each training data set comprising input training data and output training data, wherein the input training data is associated or annotated with the output training data, the output training data comprising additional training information with regard to the input training data. The additional training information can comprise a ground truth information. As a non-limiting list of examples, the ground truth information can comprise a classification information which classifies the information regarding the facial action of the patient into one of a plurality of classes, a scoring information, which associates the information regarding the facial action of the patient with a score, for example, an intensity score or a probability score, a finding information indicative of a presence or absence or intensity of a motion of the non-facial body part of the patient in the corresponding medical imaging examination. The association or annotation of the input training data with the output training data can be done manually by trained personnel and/or automatically by use or with the aid of a respective data analysis software.

In general, a trained machine learning model mimics cognitive functions that humans associate with other human minds. In particular, by training based on training data the machine learning model is able to adapt to new circumstances and to detect and extrapolate patterns. Another term for "trained machine learning model" is "trained function". In general, parameters of a machine learning model can be adapted by means of training. In particular, supervised training, semi-supervised training, unsupervised training, reinforcement learning and/or active learning can be used. Furthermore, representation learning (an alternative term is "feature learning") can be used. In particular, the parameters of the machine learning models can be adapted iteratively by several steps of training. In particular, within the training a certain cost function can be minimized. In particular, within the training of a neural network the backpropagation algorithm can be used. In particular, a machine learning model can comprise a neural network, a support vector machine, a decision tree and/or a Bayesian network, and/or the machine learning model can be based on k-means clustering, Q-learning, genetic algorithms and/or association rules. In particular, a neural network can be a deep neural network, a convolutional neural network or a convolutional deep neural network. Furthermore, a neural network can be an adversarial network, a deep adversarial network and/or a generative adversarial network.

In a further aspect, the invention relates to a computer program product comprising instructions which, when executed by a computer, cause the computer to carry out the method according to one of the aspects of the invention. In a further aspect, the invention relates to a computer-readable storage medium comprising instructions which, when executed by a computer, cause the computer to carry out the method according to one of the aspects of the invention.

The data processing system may be configured for carrying out the method according to one of the aspects of the invention. The data processing system can comprise, for example, at least one of a cloud-computing system, a distributed computing system, a computer network, a computer, a tablet computer, a smartphone or the like. The data processing system can comprise hardware and/or software. The hardware can be, for example, a processor system, a memory system and combinations thereof. The hardware can be configurable by the software and/or be operable by the software. Calculations for performing an action of a method may be carried out in the processor. Generally, all units, sub-units, or modules, in particular the calculation unit, the preprocessing unit and the camera system, may at least temporarily be in data exchange with each other, e.g., via a network connection or respective interfaces. Consequently, individual units may be located apart from each other. In particular, the data processing system may comprise the preprocessing unit.

The interface may comprise an interface for data exchange with a local server or a central web server via internet connection for receiving data and/or for providing data. The interface may be further adapted to interface with one or more users of the system, e.g., by displaying the result of the processing by the calculation unit to the user (e.g., in a graphical user interface) or by allowing the user to adjust parameters for data processing or visualization. In other words, the interface may comprise a user interface.

Data, in particular the input data and/or the camera image data, can be received, in particular received through the interface, for example, by receiving a signal that carries the data and/or by reading the data from a computer memory and/or by a manual user input, for example, through a graphical user interface. Data, in particular the patient motion data and/or the control signal, can be provided, in particular provided through the interface, for example, by transmitting a signal that carries the data and/or by writing the data into a computer memory and/or by displaying the data on a display.

Any of the algorithms, functions and/or models mentioned herein can be based on one or more of the following architectures: deep convolutional neural network, deep belief network, random forest, deep residual learning, deep reinforcement learning, recurrent neural network, Siamese network, generative adversarial network or auto-encoder.

The computer program product can be, for example, a computer program or comprise another element apart from the computer program. This other element can be hardware, for example a memory device, on which the computer program is stored, a hardware key for using the computer program and the like, and/or software, for example, a documentation or a software key for using the computer program. The computer program product may further comprise development material, a runtime system and/or databases or libraries. The computer program product may be distributed among several computer instances. A computer-readable storage medium can be embodied as non-permanent main memory (e.g. random-access memory) or as permanent mass storage (e.g. hard disk, USB stick, SD card, solid state disk).

In the context of the present invention, the expression "based on" can in particular be understood as meaning "using, inter alia". In particular, wording according to which a first feature is calculated (or generated, determined etc.) based on a second feature does not preclude the possibility of the first feature being calculated (or generated, determined etc.) based on a third feature. Reference is made to the fact that the described methods and the described systems are merely preferred example embodiments of the invention, and that the invention can be varied by a person skilled in the art, without departing from the scope of the invention as it is specified by the claims.

Features related to the invention or to the technical background of the invention will be illustrated below with reference to the accompanying figures. The illustration in the figures is schematic and highly simplified and not necessarily to scale.
Fig. 1 shows a medical imaging system.
Fig. 2 shows a face of a patient.
Fig. 3 shows a flow chart for a computer-implemented method for generating patient motion data for a medical imaging examination of a patient.
Fig. 4 shows a data processing system for generating patient motion data for a medical imaging examination of a patient.
Fig. 5 shows a flow chart for a computer-implemented method for providing a trained machine learning model.

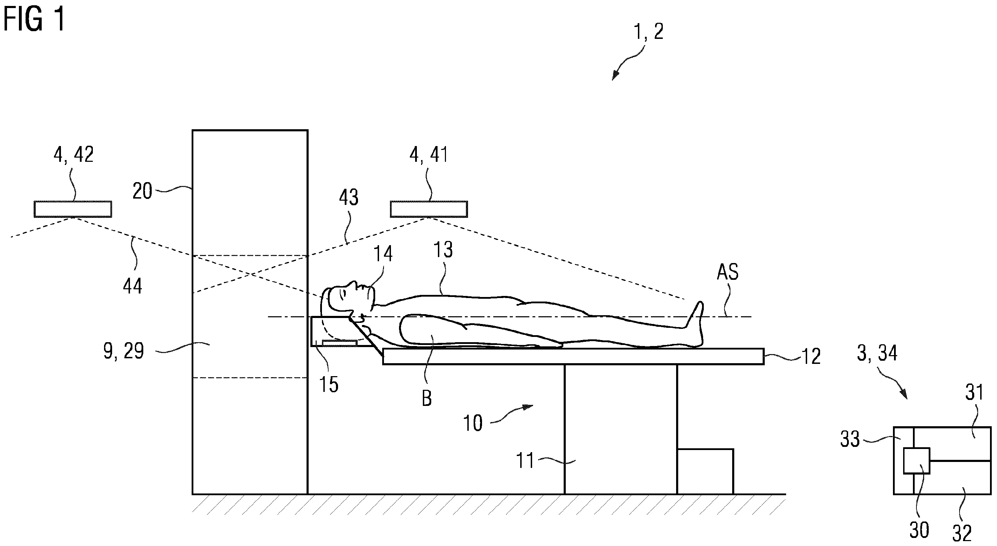

Fig. 1 shows the medical imaging system 1, comprising:
   - the data processing system 3 for generating patient motion data for a medical imaging examination of the patient 13,
   - the medical imaging device 2, configured for carrying out the medical imaging examination of the patient 13,
   - the camera system 4, configured for providing camera image data of a face F of the patient 13,
   - the preprocessing unit 30, configured for generating the input data based on the camera image data.

The data processing system 3 comprises:
- an interface 31, configured for receiving M 1 input data, the input data comprising information regarding a facial action of the patient 13, and
- a calculation unit 32, configured for applying M2 a trained machine learning model to the input data, wherein the patient motion data is generated based on the input data, the patient motion data comprising information regarding a motion of a non-facial body part B of the patient 13, wherein the information regarding the motion of the non-facial body part B of the patient 13 is generated based on the information regarding the facial action of the patient 13.

The medical imaging system comprises a patient handling system 10, configured for positioning the patient 13 relative to an examination area 29 of the medical imaging device 2 such that the face F of the patient is located in a field of view of the camera system 4 when the non-facial body part B of the patient is located in the examination area 29 of the medical imaging device 2. The data processing system 3 is implemented in form of the computer 34. The computer 34 comprises the interface 31, the calculation unit 32, the computer memory 33 and the preprocessing unit 30.

The medical imaging device 2 comprises the gantry 20 and the tunnel-shaped opening 9. The examination area 29 of the medical imaging device 2 is located in the opening 9. The patient handling system 10 is configured to insert the patient 13 into the opening 9 along the system axis AS of the medical imaging device 2. The patient handling system 10 comprises the base 11 and the board 12. The patient 13 may be inserted into the opening 9, for example, by translating the board 12 together with the patient 13 relative to the base 11 while the gantry 20 rests relative to the base 11. The patient 13 may be inserted into the opening 9, for example, by translating the gantry 20 relative to the base 11 while the board 12 and the patient 13 rest relative to the base 11.

The camera system 4 comprises the camera 41 with the field of view 43 and the camera 42 with the field of view 44. The camera 41 is located in front of the gantry 20 and above the patient handling system 10. The camera 42 is located behind the gantry 20.

According to the shown example, camera image data of a face F of the patient 13 is received, wherein the input data is generated based on the camera image data. According to the shown example, the input data is generated by applying an algorithm for face detection and/or feature extraction to the camera image data. According to the shown example, the information regarding the facial action of the patient 13 is indicative of a movement of at least one facial muscle and/or of a movement of at least one facial landmark L. According to the shown example, the information regarding the facial action of the patient 13 is indicative of a facial action unit according to a facial action coding system.

According to the shown example, the information regarding the motion of the non-facial body part B of the patient 13 is indicative of a probability that the motion of the non-facial body part B of the patient 13 will occur during the medical imaging examination of the patient 13. According to the shown example, the information regarding the motion of the non-facial body part B of the patient 13 is indicative of an impact of the motion of the non-facial body part B of the patient 13 on the medical imaging examination of the patient 13. According to the shown example, the medical imaging examination of the patient 13 by a medical imaging device 2 is adjusted based on the patient motion data. According to the shown example, the non-facial body part B of the patient 13 comprises at least a region of a torso of the patient 13 and/or at least a region of an extremity of the patient 13.

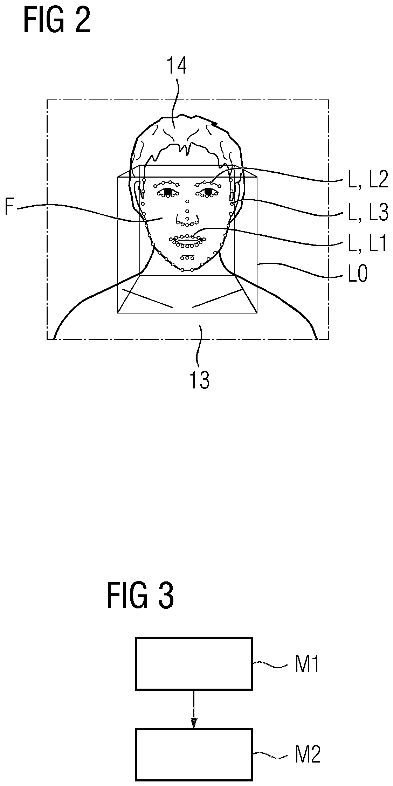

Fig. 2 shows the face F of the patient 13 with the landmarks L marking the mouth L1, the eyebrow L2 and the ear L3. The cube L0 illustrates the axes of the head 14 of the patient 13, in particular the longitudinal axis, the front-to-back axis and the left-to-right axis z. These axes may be used, for example, for face frontalization.

Fig. 3 shows a flow chart for a computer-implemented method for generating patient motion data for a medical imaging examination of a patient 13, the method comprising:
- receiving M1 input data, the input data comprising information regarding a facial action of the patient 13,
- applying M2 a trained machine learning model to the input data, wherein the patient motion data is generated based on the input data, the patient motion data comprising information regarding a motion of a non-facial body part B of the patient 13, wherein the information regarding the motion of the non-facial body part B of the patient 13 is generated based on the information regarding the facial action of the patient 13.

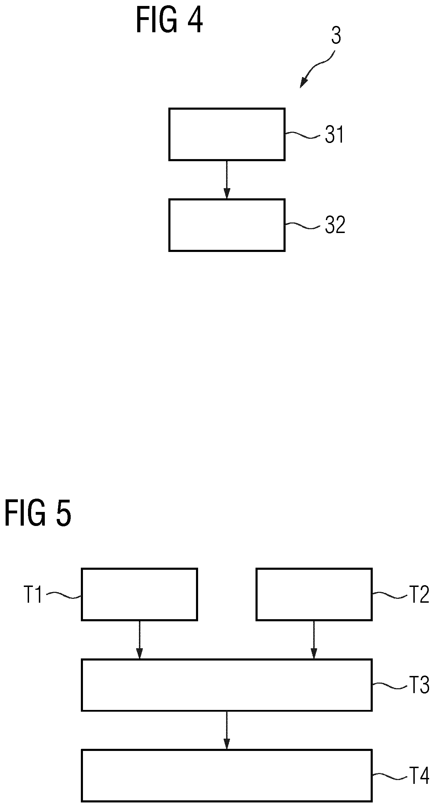

Fig. 4 shows the data processing system 3 for generating patient motion data for a medical imaging examination of the patient 13.

Fig. 5 shows a flow chart for a computer-implemented method for providing a trained machine learning model, the method comprising:
- receiving T1 input training data, the input training data comprising information regarding a facial action of a patient 13,
- receiving T2 output training data, wherein the output training data is related to the input training data, the output training data comprising information regarding a motion of a non-facial body part B of the patient 13, wherein the information regarding the motion of the non-facial body part B of the patient 13 is related to the information regarding the facial action of the patient 13,
- training T3 a machine learning model based on the input training data and the output training data,
- providing T4 the trained machine learning model.

## Claims

1. A computer-implemented method for generating patient motion data for a medical imaging examination of a patient (13), the method comprising:
- receiving (M1) input data, the input data comprising information regarding a facial action of the patient (13),
- applying (M2) a trained machine learning model to the input data, wherein the patient motion data is generated based on the input data, the patient motion data comprising information regarding a motion of a non-facial body part (B) of the patient (13), wherein the information regarding the motion of the non-facial body part (B) of the patient (13) is generated based on the information regarding the facial action of the patient (13).

2. The method according to claim 1,
- wherein camera image data of a face (F) of the patient (13) is received,
- wherein the input data is generated based on the camera image data.

3. The method according to claim 2,
- wherein the input data is generated by applying an algorithm for face detection and/or feature extraction to the camera image data.

4. The method according to one of the claims 1 to 3,
- wherein the information regarding the facial action of the patient (13) is indicative of a movement of at least one facial muscle and/or of a movement of at least one facial landmark (L).

5. The method according to one of the claims 1 to 4,
- wherein the information regarding the facial action of the patient (13) is indicative of a facial action unit according to a facial action coding system.

6. The method according to one of the claims 1 to 5,
- wherein the information regarding the motion of the non-facial body part (B) of the patient (13) is indicative of a probability that the motion of the non-facial body part (B) of the patient (13) will occur during the medical imaging examination of the patient (13).

7. The method according to one of the claims 1 to 6,
- wherein the information regarding the motion of the non-facial body part (B) of the patient (13) is indicative of an impact of the motion of the non-facial body part (B) of the patient (13) on the medical imaging examination of the patient (13).

8. The method according to one of the claims 1 to 7,
- wherein the medical imaging examination of the patient (13) by a medical imaging device (2) is adjusted based on the patient motion data.

9. The method according to one of the claims 1 to 8,
- wherein the non-facial body part (B) of the patient (13) comprises at least a region of a torso of the patient (13) and/or at least a region of an extremity of the patient (13).

10. A data processing system (3) for generating patient motion data for a medical imaging examination of a patient (13), the data processing system (3) comprising:
- an interface (31), configured for receiving (M1) input data, the input data comprising information regarding a facial action of the patient (13), and
- a calculation unit (32), configured for applying (M2) a trained machine learning model to the input data, wherein the patient motion data is generated based on the input data, the patient motion data comprising information regarding a motion of a non-facial body part (B) of the patient (13), wherein the information regarding the motion of the non-facial body part (B) of the patient (13) is generated based on the information regarding the facial action of the patient (13).

11. A medical imaging system (1), comprising:
- the data processing system (3) of claim 10,
- a medical imaging device (2), configured for carrying out the medical imaging examination of the patient (13),
- a camera system (4), configured for providing camera image data of a face (F) of the patient (13),
- a preprocessing unit (30), configured for generating the input data based on the camera image data.

12. The medical imaging system (1) according to claim 11, comprising:
- a patient handling system (10), configured for positioning the patient (13) relative to an examination area (29) of the medical imaging device (2) such that the face (F) of the patient is located in a field of view of the camera system (4) when the non-facial body part (B) of the patient is located in the examination area (29) of the medical imaging device (2).

13. A computer-implemented method for providing a trained machine learning model, the method comprising:
- receiving (T1) input training data, the input training data comprising information regarding a facial action of a patient (13),
- receiving (T2) output training data, wherein the output training data is related to the input training data, the output training data comprising information regarding a motion of a non-facial body part (B) of the patient (13), wherein the information regarding the motion of the non-facial body part (B) of the patient (13) is related to the information regarding the facial action of the patient (13),
- training (T3) a machine learning model based on the input training data and the output training data,
- providing (T4) the trained machine learning model.

14. A computer program product comprising instructions which, when executed by a computer (34), cause the computer (34) to carry out the method of one of claims 1 to 9 or the method of claim 13.

15. A computer-readable storage medium comprising instructions which, when executed by a computer (34), cause the computer (34) to carry out the method of one of claims 1 to 9 or the method of claim 13.
